# EUROPEAN PATENT APPLICATION

(11) **EP 3 756 674 A1**
(43) Date of publication of application: **30.12.2020**
(21) Application number: 20170733.8
(22) Date of filing: 28.09.2007
(51) Int. Cl.: A61K 35/12, C12N 15/113, A61K 31/7088, A61K 39/00, A61P 37/02

(54) **APOPTOTIC CELL-MEDIATED TRANSFECTION OF MAMMALIAN CELLS WITH INTERFERING RNA**

(30) Priority: 28.09.2006 US 82734306 P
(62) Document number(s) of the earlier application(s) in accordance with Art. 76 EPC: 17201450.8 / 3 351 252; 15152934.4 / 2 868 743; 12159304.0 / 2 489 729; 07843470.1 / 2 069 375
(71) Applicant: LOMA LINDA UNIVERSITY, Loma Linda, CA 92350 (US)
(72) Inventor: LI, Fengchun, Loma Linda, CA 92354-2732 (US); ESCHER, Alan P., Linda Loma, CA 92374 (US)
(74) Representative: Gregson, Anna Louise

(57) **Abstract**

Mammalian host cells for use in a cell-mediated transfection process, which contain an RNAi molecule and an expression vector for a pro-apoptotic protein. The method includes inducing apoptotic cell (AC) death in mammalian cells that contain an RNAi molecule capable of down-regulating a chosen target gene. Living cells expressing the target gene are then exposed to the ACs. The ACs are processed by the living cells, and the RNAi molecule in the ACs down-regulates the expression of the target gene in living cells.

SEQUENCE LISTING

<110>  Loma Linda University

<120>  APOPTOTIC CELL-MEDIATED TRANSFECTION OF MAMMALIAN CELLS WITH
       INTERFERING RNA

<130>  P47988EP-D4-PCT

<150>  US 60/827,343
<151>  2006-09-28

<160>  5

<170>  PatentIn version 3.5

<210>  1
<211>  62
<212>  DNA
<213>  Artificial Sequence

<220>
<223>  Synthetic oligonucleotide

<400>  1
caccgtagcg cggtgtatta taccagtgtg ctgtcctggt ataatacacc gcgctacttt      60

tt                                                                      62


<210>  2
<211>  56
<212>  DNA
<213>  Artificial Sequence

<220>
<223>  Synthetic oligonucleotide

<400>  2
gcctgtgcct cttcagctac cgaagcttgg gtagctgaag aggcacaggc tttttt          56


<210>  3
<211>  577
<212>  DNA
<213>  Homo sapiens

<400>  3
atggacgggt ccggggagca gcccagaggc gggggcccca ccagctctga gcagtcatga      60

agacaggggc ccttttgctt cagggtttca tccaggatcg agcagggcga agggggggga      120

ggcacccgag ctggccctgg acccggtgcc tcaggatgcg tccaccaaga agctgagcga     180

gtgtctcaag cgcatcgggg acgaactgga cagtaacatg gagctgcaga ggatgattgc     240

cgccgtggac acagactccc cccgagaggt ctttttccga gtggcagctg acatgttttc     300

tgacggcaac ttcaactggg gccgggttgt cgcccttttc tactttgcca gcaaactggt     360

gctcaaggcc ctgtgcacca aggtgccgga actgatcaga accatcatgg gctggacatt     420

ggacttcctc cgggagcggc tgttgggctg gatccaagac cagggtggtt gggacggcct     480

cctctcctac tttgggacgc ccacgtggca gaccgtgacc atctttgtgg cgggagtgct 540

caccgcctcg ctcaccatct ggaagaagat gggctga 577

<210> 4
<211> 1653
<212> DNA
<213> Photinus pyralis

<400> 4
atggaagacg ccaaaaacat aaagaaaggc ccggcgccat tctatccgct ggaagatgga 60

accgctggag agcaactgca taaggctatg aagagatacg ccctggttcc tggaacaatt 120

gcttttacag atgcacatat cgaggtggac atcacttacg ctgagtactt cgaaatgtcc 180

gttcggttgg cagaagctat gaaacgatat gggctgaata caaatcacag aatcgtcgta 240

tgcagtgaaa actctcttca attctttatg ccggtgttgg gcgcgttatt tatcggagtt 300

gcagttgcgc ccgcgaacga catttataat gaacgtgaat tgctcaacag tatgggcatt 360

tcgcagccta ccgtggtgtt cgtttccaaa aaggggttgc aaaaaatttt gaacgtgcaa 420

aaaaagctcc caatcatcca aaaaattatt atcatggatt ctaaaacgga ttaccaggga 480

tttcagtcga tgtacacgtt cgtcacatct catctacctc ccggttttaa tgaatacgat 540

tttgtgccag agtccttcga tagggacaag acaattgcac tgatcatgaa ctcctctgga 600

tctactggtc tgcctaaagg tgtcgctctg cctcatagaa ctgcctgcgt gagattctcg 660

catgccagag atcctatttt tggcaatcaa atcattccgg atactgcgat tttaagtgtt 720

gttccattcc atcacggttt tggaatgttt actacactcg gatatttgat atgtggattt 780

cgagtcgtct taatgtatag atttgaagaa gagctgtttc tgaggagcct tcaggattac 840

aagattcaaa gtgcgctgct ggtgccaacc ctattctcct tcttcgccaa aagcactctg 900

attgacaaat acgatttatc taatttacac gaaattgctt ctggtggcgc tcccctctct 960

aaggaagtcg gggaagcggt tgccaagagg ttccatctgc caggtatcag gcaaggatat 1020

gggctcactg agactacatc agctattctg attacacccg agggggatga taaaccgggc 1080

gcggtcggta aagttgttcc attttttgaa gcgaaggttg tggatctgga taccgggaaa 1140

acgctgggcg ttaatcaaag aggcgaactg tgtgtgagag gtcctatgat tatgtccggt 1200

tatgtaaaca atccggaagc gaccaacgcc ttgattgaca aggatggatg gctacattct 1260

ggagacatag cttactggga cgaagacgaa cacttcttca tcgttgaccg cctgaagtct 1320

ctgattaagt acaaaggcta tcaggtggct cccgctgaat tggaatccat cttgctccaa 1380

caccccaaca tcttcgacgc aggtgtcgca ggtcttcccg acgatgacgc cggtgaactt 1440

cccgccgccg ttgttgtttt ggagcacgga aagacgatga cggaaaaaga gatcgtggat 1500

tacgtcgcca gtcaagtaac aaccgcgaaa aagttgcgcg gaggagttgt gtttgtggac 1560

gaagtaccga aaggtcttac cggaaaactc gacgcaagaa aaatcagaga gatcctcata 1620

aaggccaaga agggcggaaa gatcgccgtg taa 1653

<210> 5
<211> 936
<212> DNA
<213> Renilla reniformis

<400> 5
atgacttcga aagtttatga tccagaacaa aggaaacgga tgataactgg tccgcagtgg 60

tgggccagat gtaaacaaat gaatgttctt gattcattta ttaattatta tgattcagaa 120

aaacatgcag aaaatgctgt tattttttta catggtaacg cggcctcttc ttatttatgg 180

cgacatgttg tgccacatat tgagccagta gcgcggtgta ttataccaga tcttattggt 240

atgggcaaat caggcaaatc tggtaatggt tcttataggt tacttgatca ttacaaatat 300

cttactgcat ggtttgaact tcttaattta ccaaagaaga tcatttttgt cggccatgat 360

tggggtgctt gtttggcatt tcattatagc tatgagcatc aagataagat caaagcaata 420

gttcacgctg aaagtgtagt agatgtgatt gaatcatggg atgaatggcc tgatattgaa 480

gaagatattg cgttgatcaa atctgaagaa ggagaaaaaa tggttttgga gaataacttc 540

ttcgtggaaa ccatgttgcc atcaaaaatc atgagaaagt tagaaccaga agaatttgca 600

gcatatcttg aaccattcaa agagaaaggt gaagttcgtc gtccaacatt atcatggcct 660

cgtgaaatcc cgttagtaaa aggtggtaaa cctgacgttg tacaaattgt taggaattat 720

aatgcttatc tacgtgcaag tgatgattta ccaaaaatgt ttattgaatc ggatccagga 780

ttcttttcca atgctattgt tgaaggcgcc aagaagtttc ctaatactga atttgtcaaa 840

gtaaaaggtc ttcatttttc gcaagaagat gcacctgatg aaatgggaaa atatatcaaa 900

tcgttcgttg agcgagttct caaaaatgaa caataa 936

## Description

### CROSS-REFERENCE TO RELATED APPLICATIONS

The present application claims the benefit of United States Provisional Patent Application 60/827,343, titled "Apoptotic cell-mediated transfection of mammalian cells with interfering RNA," filed September 28, 2006; the contents of which are incorporated in this disclosure by reference in their entirety.

### BACKGROUND

RNA interference (RNAi) is a mechanism in molecular biology where the presence of certain fragments of double-stranded RNA (dsRNA) interferes with the expression of a particular gene, which shares a homologous sequence with the dsRNA. RNAi is a gene silencing process that requires active participation of cellular machinery. Although the specific mechanism is poorly understood, it is known that the ribonuclease enzyme Dicer binds to and cleaves short double-stranded RNA molecules (dsRNA) to produce double-stranded fragments of 21-23 base pairs with two-base single-stranded overhangs on each end. The short double-stranded fragments produced by Dicer, called small interfering RNAs (siRNAs), are then separated, presumably by an enzyme with helicase activity, and integrated into a multiprotein complex called the RNA-induced silencing complex (RISC).

Synthetic siRNAs and short hairpin RNAs (shRNAs) can be designed to have identical function. Whereas, siRNA are 2 strands of complementary RNA that can be synthesized, a shRNA is encoded by DNA as a single RNA molecule that hybridze to itself with a loop at one end. The loop is then cleaved intracellularly yielding a molecule similar to a siRNA. There are thousands of RNAi sequences available that are capable of downregulating gene expression. (See, e.g. Behlke, 2006, Mol Ther vol. 13 p644). This method has become a universally accepted means of downregulating expression of any gene in mammalian cells.

Presently, RNAi molecules are delivered via electroporation, cationic- and liposome-mediated transfection, viral delivery, and direct injection (Behlke, 2006, Mol Ther vol. 13 p644). One group has shown that bacteria can be used to deliver RNAi molecules to mammalian cells to screen for targeting siRNA molecules (Zhao et al., 2005, Nat Methods vol 2 p967).

Antigen-presenting cells (APCs) like dendritic cells (DCs) are a major target for manipulation of immune responses and they have been modified using RNAi (Li et al., 2004, Immu Res vol 30 p215). However, there is no available method that permits guaranteed co-delivery of multiple antigens and RNAi molecules to the same APC.

### SUMMARY

The invention utilizes apoptotic cells (ACs) for the delivery to living cells of short RNAs capable of downregulating gene expression via RNA interference (RNAi). The invention addresses the problem of delivering RNAi molecules to mammalian cells *in vivo,* and the ability to link presence of an already synthesized antigen(s) with an RNAi molecule as part of the same package to be delivered.

In one embodiment the invention provides a method of generating ACs containing an RNAi molecule, which includes the steps of (1) providing an RNAi molecule, such as short interfering RNA (siRNA) or a vector capable of expressing a short hairpin RNA (shRNA), directed to a target gene of interest; (2) introducing the RNAi molecule into a pre-apoptotic cells (pre-ACs), preferably by transfection; and (3) inducing apoptosis, e.g., by UV exposure or expression of a pro-apoptotic protein like BAX, to create an AC containing the RNAi molecule.

In one embodiment the RNAi molecule contains a polynucleotide sequence substantially complementary to a messenger RNA (mRNA) encoding the target gene. In a preferred embodiment the RNAi molecule comprises a double-stranded RNA (dsRNA), which contains a sense sequence corresponding a partial sequence of the target gene mRNA and an antisense sequence that is substantially complementary and capable of specifically hybridizing to a target gene mRNA

In one embodiment the RNAi molecule comprises a short double-stranded RNA molecule (dsRNA) of about 19-27 base pairs. In a preferred embodiment, the RNAi molecule is a siRNA, comprising a short double-stranded RNA molecule (dsRNA) of about 19-23 base pairs, each strand having a single-stranded overhang of about two bases on one end.

In another embodiment, the RNAi molecule is provided by a vector capable of expressing a short hairpin RNA (shRNA) or a short interfering RNA (siRNA). In a preferred embodiment, the vector contains one or more than one RNA polymerase III promoter controlling transcription of the RNAi molecule.

In one embodiment, the RNAi molecule is introduced into the mammalian cell by transfection, electroporation or microinjection. In another embodiment, the RNAi molecule is introduced into the mammalian cell by delivering a DNA plasmid or viral vector encoding a short hairpin RNA (shRNA).

In one embodiment, the method includes the further step of introducing a plasmid DNA or viral expression vector containing a polynucleotide sequence encoding a pro-apoptotic protein, such as BAX protein, into the pre-apoptotic mammalian cells.

In one embodiment the RNAi molecule and the expression vector containing a polynucleotide sequence encoding a pro-apoptotic protein are both introduced into the mammalian cell, e.g. by co-transfection *in vitro* or by introducing the RNAi molecule and expression vector into an organ or tissue by electroporation, gene-gun, or injection.

In one embodiment, the present invention provides a method of transfecting a mammalian cell, which includes the steps of: (a) providing a mammalian cell expressing a target gene, wherein the mammalian cell is capable of phagocytosis; and (b) exposing the mammalian cell to an apoptotic cell, containing an RNAi molecule capable of downregulating the target gene, under conditions whereby the apoptotic cell is taken up by the mammalian cell. The RNAi molecule then downregulates expression of the target gene in the mammalian cell. In alternative embodiments, the mammalian cells are exposed to the apoptotic cells *in vivo* or *in vitro.* In a preferred embodiment, the mammalian cell is an antigen presenting cell.

In another embodiment, the present invention provides a mammalian host cell, comprising: (a) One or several RNAi molecules capable of downregulating a target gene; and (b) an expression vector capable of expressing a pro-apoptotic protein. In a preferred embodiment the mammalian host cell expresses one or several antigens, like autoantigens or donor antigens. Mammalian host cells in accordance with this aspect of the present invention can be converted to ACs for use in cell-mediated transfection procedures.

Many cells can process ACs, in particular, antigen presenting cells (APCs) like dendritic cells (DCs) that direct immune responses. The ability to deliver antigen and a RNAi molecule capable of modifying the function of an APC, like DC, as part of the same package will permit increased control over induced immune responses (i.e., tolerogenic vs immunogenic) for antigens present in ACs. This approach can be adapted for use in prevention of transplant rejection (with donor antigens) and treatment of autoimmune diseases (with autoantigens).

### BRIEF DESCRIPTION OF THE DRAWINGS

These features, aspects and advantages of the present invention will become better understood with regard to the following description, appended claims and accompanying drawings where:
Figure 1 shows schematic depictions of the plasmids used to generate mammalian cells containing an RNAi molecule (shRUC and shII) and/or to generate ACs (BAX), as well as plasmids containing reporter genes (RUC and LUC) used to monitor the downregulation of a target gene (RUC) in accordance with a method of the present invention;
Figure 2 shows Renilla luciferase (RUC) activity from COS-7 cells expressing the RUC cDNA and co-cultured with differently treated COS-7 ACs;
Figure 3 shows the effects of duration of expression of shRUC prior to induction of apoptosis on Renilla luciferase activity in live cells; and
Figure 4 shows the effects of UV- and BAX-induced ACs containing shRUC on RUC mRNA levels expressed by live cells.

### DETAILED DESCRIPTION

According to one embodiment of the present invention, there is provided a method for generating an apoptotic cell (AC) that contains an interfering RNA (RNAi) molecule capable of down regulating a chosen target gene. According to another embodiment of the present invention, there is provided method for delivering the RNAi molecule to a mammalian cell expressing the target gene using the AC. According to another embodiment of the present invention, there is provided a mammalian host cell containing an RNAi molecule and a vector capable of expressing a pro-apoptotic protein.

As used in this disclosure, except where the context requires otherwise, the term "comprise" and variations of the term, such as "comprising," "comprises" and "comprised" are not intended to exclude other additives, components, integers or steps.

As used in this disclosure, the term "substantially complementary" and variations of the term, such as "substantial complement," means that at least 90% of all of the consecutive residues in a first strand are complementary to a series of consecutive residues of the same length of a second strand. As will be understood by those with skill in the art with reference to this disclosure, one strand can be shorter than the other strand and still be substantially complementary. With respect to the invention disclosed in this disclosure, for example, the RNAi, siRNA or shRNA can be shorter or longer than the complementary messenger RNA (mRNA) for the target gene interest; however, it is preferable that the RNAi molecule is shorter than and substantially complementary to its corresponding mRNA.

One step of the method is providing an RNAi molecule directed to a target gene of interest.

"RNAi molecule" refers to a nucleic acid that forms a double stranded RNA, which double stranded RNA has the ability to reduce or inhibit expression of a gene or target gene when the RNAi molecule present in the same cell as the gene or target gene. In general, RNAi molecules are fragments of double-stranded RNA (dsRNA), which share a homologous sequence with a target gene. The dsRNA of an RNAi molecule typically contains a "sense" sequence corresponding a partial sequence of the target gene messenger RNA (mRNA) and an "antisense" sequence that is substantially complementary and capable of specifically hybridizing to a target gene mRNA.

RNAi molecules include small interfering RNAs (siRNAs), which are comprised of short dsRNA molecules. In one embodiment, a siRNA comprises a dsRNA containing an antisense sequence substantially or completely complementary to a target gene mRNA. The portions of the siRNA that hybridize to form the dsRNA are typically substantially or completely complementary to each other. The sequences of the siRNA can correspond to the full length target gene, or a subsequence thereof. Typically, the siRNA is at least about 15-50 nucleotides in length (e.g., each complementary sequence of the double stranded siRNA is 15-50 nucleotides in length, and the double stranded siRNA is about 15-50 base pairs in length), preferably about 19-27 base pairs in length, e.g., 19, 20, 21, 22, 23, 24, 25, 26 or 27 nucleotides in length.

In a preferred embodiment, the double stranded portion of the siRNA is about 19-23 base pairs and contains two-base single-stranded overhangs on each end, mimicking the product naturally produced by the endoribonuclease Dicer *in vivo.* Suitable siRNAs are integrated into a multiprotein complex called the RNA-induced silencing complex (RISC), which initiates the degradation of homologous mRNA.

Synthesis of the siRNA can readily be accomplished by phosphoramidite chemistry and can be obtained from a number of commercial sources well known in the art, as will be understood by those with skill in the art with reference to this disclosure.

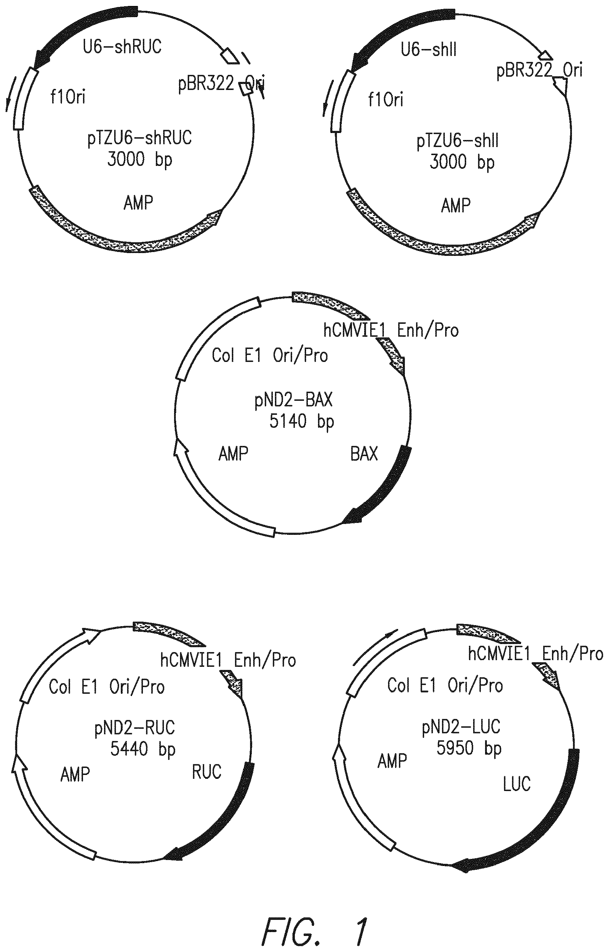

An alternative to individual chemical synthesis of siRNA is to construct a sequence for insertion in an expression vector. Several RNAi vectors for the transcription of inserts are commercially available (e.g., Ambion, Austin, TX; Invitrogen, Carlsbad, CA). Some use an RNA polymerase III (Pol III) promoter to drive expression of both the sense and antisense strands separately, which then hybridize *in vivo* to make the siRNA. Other vectors are based on the use of Pol III to drive expression of short "hairpin" RNAs (shRNA), individual transcripts that adopt stem-loop structures, which are processed into siRNAs by the RNAi machinery. An example of an RNAi vector is the pTZU6 vector shown in Figure 1.

Accordingly, RNAi molecules also include short "hairpin" RNA (shRNA), which functions in a similar manner as siRNA. Whereas siRNA is comprised of two strands of complementary RNA that can be synthesized, a shRNA is encoded by DNA as a single RNA molecule that hybridizes to itself with a loop at one end. The "hairpin" loop of the shRNA is cleaved intracellularly yielding a molecule similar to a siRNA.

A typical shRNA vector design incorporates two inverted repeats, containing the sense and antisense target sequences, separated by a loop sequence. Commonly used loop sequences contain 8-9 bases. A terminator sequence consisting of 5-6 poly dTs may be present at the 3' end and cloning sequences can be added to the 5' ends of the complementary oligonucleotides. Referring to Figure 1, two specific inserts encoding are shown, shRUC and shII, which encode shRNAs. The polynucleotide sequences for these inserts are SEQ ID NO:1 and SEQ ID NO:2.

Any gene expressed within living cells, which are capable of phagocytosis and uptake of apoptotic cells, can be selected as the target gene. For example, one could deliver plasmid DNA that expresses a RNAi molecule that regulates immunity, e.g., by downregulation of CD40 expression to induce tolerance. One or several RNAi molecules can be designed to downregulate the expression of one or several chosen target genes in living cells following a routinely used method, such as computer software or random selection of target sequence within the messenger RNA of the target gene followed by experimental determination of target RNA degradation.

Downregulation is the process by which a cell decreases the number of a cellular component, such as RNA or protein in response to external variable. RNAi down regulates a gene function by mRNA degradation. Thus, the degree of RNA interference achieved is directly proportional to the level of mature mRNA and the translated proteins. The terms "downregulate," "downregulation," "downregulating" or "downregulated" interchangeably refer to a protein or nucleic acid (RNA) that is transcribed or translated at a detectably lower level, in comparison to a normal or untreated cell. Downregulation can be detected using conventional techniques for detecting and/or measuring target mRNA (*i.e*., RT-PCR, PCR, hybridization) or target proteins (*i.e*., ELISA, immunohistochemical techniques, enzyme activity). Downregulation can be 10%, 20%, 30%, 40%, 50%, 60%, 70%, 80%, 90% etc. in comparison to a normal or untreated cell. In certain instances, downregulation is 1-fold, 2-fold, 3-fold, 4-fold or more lower levels of transcription or translation in comparison to a normal or untreated cell.

Another step of the method is introducing an RNAi molecule into a cell, which has not undergone apoptosis, i.e., a pre-apoptotic cell (pre-AC). Any mammalian cell can be used because they can be all induced to undergo apoptosis and are capable of carrying out RNAi reactions. The RNAi molecules are delivered into living cells that will be made apoptotic either *in vitro* or directly *in vivo,* depending on the desired application.

In one embodiment the pre-ACs express known or unknown antigens capable of eliciting an immune response. For example, the specific antigen may be autoantigen that is recognized by the immune system of patients suffering from a specific autoimmune disease.

The RNAi molecules can be delivered directly as RNA by transfecting cells with short interfering RNAs (siRNAs) using electroporation or other accepted methods described in the literature. For example, delivery of siRNA directly in cells can be achieved by using microinjection or the use of transfection reagent specialized for siRNA-delivery.

Alternatively, the preferred method is to deliver a DNA expression vector encoding a short hairpin RNA (shRNA) that functions as a RNAi molecule, delivered via electroporation, cationic- or liposome-mediated transfection, viral delivery, or direct injection. This approach permits higher concentrations of RNAi molecules in ACs.

After introducing the RNAi molecule into the pre-apoptotic cell, the next step of the method is inducing apoptosis, thereby creating an AC containing the RNAi molecule

As will be appreciated by one of skill in the art, apoptosis is a form of cell death in which a programmed sequence of events leads to the elimination of cells without releasing harmful substances into the surrounding area. Apoptosis plays a crucial role in developing and maintaining health by eliminating old cells, unnecessary cells, and unhealthy cells. The human body replaces perhaps a million cells a second. Apoptosis is also called programmed cell death or cell suicide. Strictly speaking, the term apoptosis refers only to the structural changes cells go through, and programmed cell death refers to the complete underlying process, but the terms are often used interchangeably.

Morphological features associated with cells undergoing apoptosis include, membrane blebbing, aggregation of chromatin at the nuclear membrane, shrinking of the cytoplasm and condensation of the nucleus, fragmentation of the cell into smaller bodies, formation of apoptotic bodies, and pore formation in the mitochondrial membrane, involving proteins of the bcl-2 family. Biochemical features associated with the energy (ATP)-dependent process of programmed cell death include non-random mono- and oligonucleosomal length fragmentation of DNA (ladder pattern after agarose gel electrophoresis), release of cytochrome c, apoptosis-inducing factor (AIF) and other factors into the cytoplasm by mitochondria, activation of the caspase cascade, and alterations in membrane biochemistry (i.e. translocation of phosphatidylserine from the cytoplasmic to the extracellular side of the membrane).

Apoptosis can be induced experimentally by exposing cells to various stimuli, including chemicals or radiation. Topoisomerase inhibitors such as etoposide (also known as VP-16) are potent inducers of apoptosis, and are widely used in the study of programmed cell death. Alternatively, cells transfected *in vitro* can be made apoptotic using exposure to ultra violet light or co-delivery of a gene or cDNA coding for a pro-apoptotic protein, for example, the BAX protein. For UV induced apoptosis, cells are simply exposed to UV-B light for 10 min at a distance of 10 cm. For BAX-induced apoptosis, delivery and expression of the cDNA into cells is sufficient to trigger apoptosis.

In one embodiment, the method includes the further step of introducing a plasmid DNA or viral expression vector containing a polynucleotide sequence encoding a pro-apoptotic protein into the mammalian cells. With reference to Figure 1, there is shown a map for such vector, pND2-BAX, wherein expression of the BAX cDNA is under the control of the hCMV IE1 enhancer/promoter. The polynucleotide sequence encoding the BAX protein is set forth in SEQ ID NO:3.

Cells can be transfected *in vitro,* made apoptotic and then injected into a patient, preferably intravenously. A similar approach can be used to generate ACs containing RNAi molecules *in vivo.* In this case the preferred approach is to deliver plasmid DNA coding for shRNA of choice as well as a pro-apoptotic protein. The DNA can be delivered into a chosen organ or tissue, using electroporation, gene-gun, or injection.

In one embodiment the invention further provides a method of transfecting mammalian cells by exposing a live cell containing a target gene to an AC containing an RNAi molecule directed to the target gene so that the RNAi molecule downregulates expression of the target gene.

The live mammalian cells can be cell lines grown *in vitro,* or cells of any given tissue in a living body *in vivo.* Living cells expressing one or several genes targeted by the RNAi molecule gene are exposed to the ACs containing the RNAi molecule. Any endogenous or exogenous gene expressed within living cells can be the target of the RNAi molecule. Expression of an exogenous gene can be accomplished by introduction of an expression vector containing a polynucleotide encoding a target gene of interest. Again, these cells can be cells grown *in vitro* or can be cells of any tissue *in vivo.*

The *in vitro* experiments disclosed herein demonstrate that RNAi molecules present in ACs can transfect living cells with the RNAi molecules. The ACs are phagocytosed and processed by the living cells, and the RNAi molecules that were present in the ACs downregulate the expression of the target gene(s) in living cells.

Most cells have some phagocytic ability, however, the two most important cell types whose major function is phagocytosis are polymorphonuclear leukocytes and the monocyte-macrophage lineage cells (monocytes, macrophages, Kupffer cells, Langerhans cells, dendritic cells, and glial cells). As will be appreciated by one of skill in the art, phagocytosis of ACs occurs constantly *in vivo* to remove dead cells. Accordingly, it is expected that phagocytosis and uptake of ACs containing RNAi molecules will also occur *in vivo,* as has been shown for ACs carrying genomic DNA. (Holmgren et al, 1999, Blood vol 11 p3956)

Many cells can process ACs, in particular, antigen-presenting APCs, like DCs, that direct immune responses. An antigen-presenting cell (APC) is a cell that displays foreign antigen complexed with MHC on its surface. T-cells may recognize this complex using their T-cell receptor (TCR). Although almost every cell in the body is technically an APC, since it can present antigen to CD8+ T cells via MHC class I molecules, the term is often limited to those specialized cells that can prime T cells (i.e., activate a T cell that has not been exposed to antigen. These cells generally express MHC class II as well as MHC class I molecules, and can stimulate CD4+ ("helper") cells as well as CD8+ ("cytotoxic") T cells. Traditional antigen-presenting cells include macrophages; dendritic cells; Langherhans cells; and B-lymphocytes. Other cells, like fibroblasts (skin), thymic epithelial cells, thyroid epithelial cells, glial cells (brain), pancreatic beta cells and vascular endothelial cells, can be stimulated by certain cytokines such as IFN-γ, to express the major histocompatibility complex proteins required for interaction with naive T cells.

A significant advantage of AC-mediated transfection of APCs with RNAi molecules is that it will permit the co-delivery of any and all antigens present in ACs together with one or possibly several selected RNAi molecules to the same APCs. In addition, AC-mediated transfection is a physiological means of delivering RNAi that could result in a high number of transfected cells, because ACs are rapidly phagocytosed and recruit APCs *in vivo.*

The ability to deliver antigen and a RNAi molecule capable of modifying the function of APCs, like DCs, as part of the same package permits increased control over induced immune responses (i.e., tolerogenic vs immunogenic) for antigens present in ACs. Important applications for this approach include the prevention of transplant rejection (with donor antigens) and treatment of autoimmune diseases (with autoantigens).

The clinical potential applications of this approach are multiple, and include any situation where a gene must be downregulated for therapeutic purposes. The approach is particularly well-suited for manipulation of immune responses because antigen-presenting cells are very efficient at taking in and processing ACs. The ability to deliver antigen(s) and RNAi molecules as a single package means that a specific dendritic cell will mount an immune response directed by the RNAi molecules to the antigen(s) of the ACs. For example, if one wishes to induce tolerance or immunity to a specific antigen, one could deliver plasmid DNA coding the antigen, a RNAi molecule that regulates immunity, for example downregulation of CD40 expression to induce tolerance, and a pro-apoptotic protein. Such ACs would be processed by APCs which would be more likely to trigger tolerance for the antigen(s) carried by ACs.

The invention provides for the generation of mammalian ACs containing a chosen RNAi molecule that downregulates the expression of a chosen target gene. The ACs can be generated using UV or a pro-apoptotic cDNA like that coding for the BAX protein. The invention may be appreciated in certain aspects with reference to the following examples, offered by way of illustration, not by way of limitation. Materials, reagents and the like to which reference is made in the following examples are obtainable from commercial sources, unless otherwise noted.

Figure 1 shows schematic depictions of the plasmids used to generate mammalian cells containing an RNAi molecule (shRUC and shII) and/or to generate ACs (BAX), as well as plasmids containing reporter genes (RUC and LUC) used to monitor the downregulation of a target gene (RUC) in accordance with a method of the present invention. The plasmid maps were prepared using **Plasmid Processor W software** (T. Kivirauma, P. Oikari and J.Saarela, Dept. of Biochemistry & Biotechnology, U. of Kuopio, plasmid@uku.fi, Home: http://www.uku.fi/~kiviraum/plasmid/plasmid.html, Archive: iubio/ibmpc/plasmid-processor*, ebi/dos/plasmid).

Referring to Figure 1, the sequence of shRUC for Renilla luciferase site C introduced into the pTZU6-shRUC plasmid is SEQ ID NO:1. The sequence of shII for HIV-1 rev (site II) introduced into the pTZU6-shII plasmid is SEQ ID NO:2. The sequence of BAX for human BAX inserted into the pND2-BAX plasmid is SEQ ID NO:3. The sequence of LUC for Firefly luciferase inserted into the pND2-LUC plasmid is SEQ ID NO:4. The sequence of RUC for Renilla luciferase introduced into the pND2-RUC plasmid is SEQ ID NO:5.

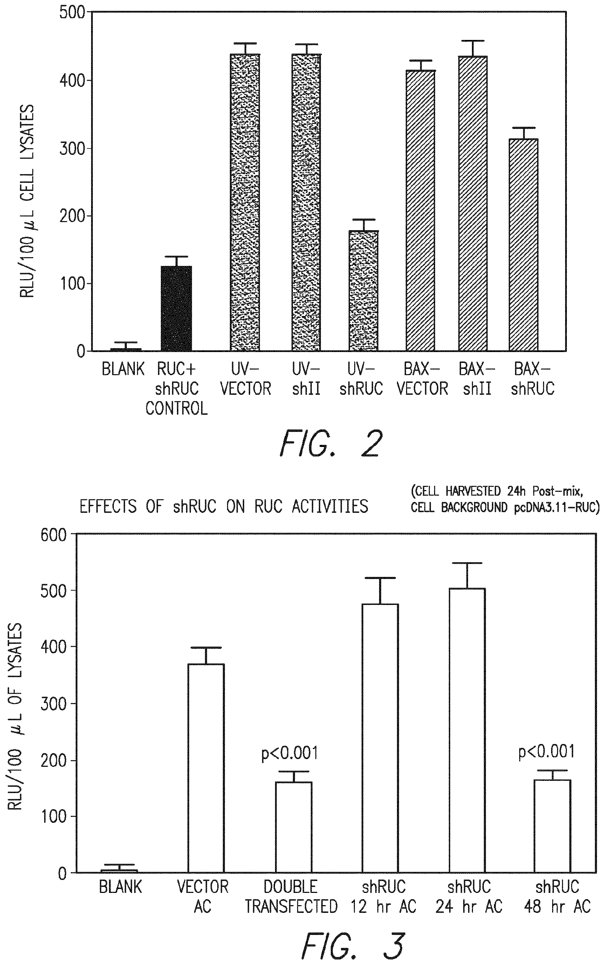

As an example, Figure 2 shows the effect of ACs containing a short hairpin RNA (shRUC) that causes degradation of the Renilla luciferase mRNA. Simian COS-7 cells expressing Renilla luciferase cDNA were incubated with UV- or BAX-induced apoptotic COS-7 cells containing shRUC, and Renilla luciferase activity was measured.

COS-7 cells were transfected with 5 µg RUC plasmid DNA coding for Renilla luciferase to measure effects of ACs and 2 µg LUC plasmid DNA coding for firefly luciferase for normalization. Differently treated COS-7 cells were made apoptotic and added to the live COS-7 cells 3 hours after the live cells had been transfected with luciferase. UV- and BAX-induced apoptosis yielded ∼80% and ∼30% ACs, respectively. The ratio of cells induced to be apoptotic added to living cells expressing luciferase cDNA was 2:1. Cells were then harvested after 20 hours culture to measure luciferase activities. Staining of live and apoptotic COS-7 cells showed uptake of ACs by live cells (data not shown).

All transfections were performed using Superfect (Qiagen, Valencia, CA). Measurements were performed in triplicate from 2 separate experiments.

Figure 2 shows Renilla luciferase (RUC) activity from COS-7 cells expressing the RUC cDNA and co-cultured with differently treated COS-7 ACs. Referring now to Figure 2: Blank shows background luminescence activity from untransfected cells; RUC+shRUC control: shows RUC activity when cells were co-transfected with luciferase plasmids (5 µg RUC, 2 µg LUC) and plasmid encoding shRUC (10 µg) to confirm downregulating activity of shRUC (no ACs added); UV-Vector shows RUC activity when added ACs were generated by transfecting COS-7 cells with 10 µg plasmid vector alone and UV exposure 48 hrs post transfection; UV-shII shows RUC activity when the pre-ACs were transfected with 10 µg plasmid DNA encoding a shRNA targeting the HIV virus II gene as negative control and made apoptotic as described for UV-AC vector; UV-AC shRUC shows RUC activity when the pre-ACs were transfected with 10 µg plasmid DNA encoding a shRNA targeting the RUC cDNA and made apoptotic as described for UV-AC vector; BAX-vector shows RUC activitiy when the pre-ACs were co-transfected with plasmid DNA coding for BAX (10 µg) and plasmid vector alone (10 µg) and ACs were harvested 30 hrs post transfection (no UV-treatment); BAX-shII shows RUC activity when the pre-ACs were transfected with plasmid DNA coding for BAX and control shRNA and processed as described for BAX-vector; and BAX-shRUC shows RUC activity when the pre-ACs were transfected with plasmid DNA coding for BAX and shRUC and processed as described for BAX-vector.

These results show ACs containing shRUC decreased luciferase activity in live cells expressing an RUC target gene. In contrast, co-cultivation with ACs containing a control shRNA (shII) targeting the HIV-1 *rev* gene did not. Addition of ACs containing vector alone did not affect Renilla luciferase activity (data not shown).

Figure 3 shows the effects of duration of expression of shRUC prior to induction of apoptosis of shRUC-containing cells on Renilla luciferase activity in live cells. The data indicate that ACs containing shRUC that had been expressed for 12 and 24 hrs did not downregulate activity of luciferase after incubating the apopotic and live cells. Expression of shRUC for 48 hrs was necessary to observe loss of luciferase actvity. These data indicate that the loss of luciferase activity after adding ACs containing shRUC was not due to shRUC plasmid contamination into cells expressing RUC luciferase cDNA, but to expression of shRUC contained by ACs.

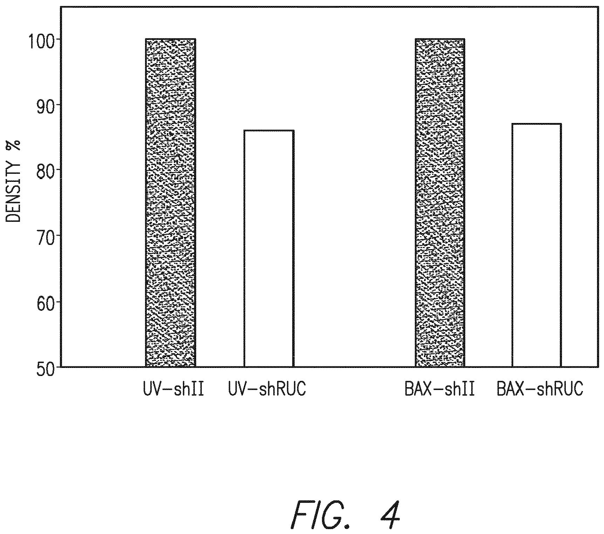

Figure 4 shows the effects of UV- and BAX-induced ACs containing shII or shRUC on levels of Renilla luciferase mRNA in live cells exposed to the AC. Live COS-7 cells transfected with luciferase cDNA were co-cultured with COS-7 ACs containing control shRNA (shII) or shRNA targeting RUC mRNA (shRNA), and induced with UV or BAX, as described for Figure 2. Total RNA was isolated and semi-quantitative RT-PCR was performed with 100, 200 and 400 ng total RNA template using primers for RUC and the housekeeping gene GAPD-H. Products were separated using agarose gel electrophoresis and cDNA band densities were determined. RUC cDNA amount was normalized for GAPD-H cDNA amount when comparing shII and shRUC treatments for a given method of apoptosis induction. Data is shown as percentage of RUC cDNA found in shRUC-treated cells compared to shII-treated cells.

These results show that shRUC contained by ACs decreased RUC mRNA levels in live cells exposed to the ACs.

The invention includes the following embodiments.
1. A method of generating an apoptotic cell containing an RNAi molecule comprising:
   (a) providing an RNAi molecule, wherein the RNAi molecule is capable of downregulating a target gene of interest;
   (b) introducing the RNAi molecule into a mammalian cell; and
   (c) inducing apoptosis to create an apoptotic cell containing the RNAi molecule.
2. The method of embodiment 1 wherein the RNAi molecule is a short interfering RNA (siRNA) or a short hairpin RNA (shRNA).
3. The method of embodiment 1, wherein the RNAi molecule contains a polynucleotide sequence substantially complementary to a messenger RNA (mRNA) encoding the target gene.
4. The method of embodiment 3, wherein the RNAi molecule comprises a double-stranded RNA (dsRNA), which contains a sense sequence corresponding a partial sequence of the target gene mRNA and an antisense sequence that is substantially complementary and capable of specifically hybridizing to a target gene mRNA.
5. The method of embodiment 1 wherein the RNAi molecule comprises a short double-stranded RNA molecule (dsRNA) of about 19-27 base pairs.
6. The method of embodiment 2 wherein the siRNA is a short double-stranded RNA molecule (dsRNA) of about 19-23 base pairs, each strand having a single-stranded overhang of about two bases on one end.
7. The method of embodiment 1, wherein the RNAi molecule is provided by a vector capable of expressing a short hairpin RNA (shRNA) or a short interfereing RNA (siRNA).
8. The method of embodiment 7, wherein the vector contains one or more than one RNA polymerase III promoter controlling transcription of the RNAi molecule.
9. The method of embodiment 1, where the RNAi molecule is introduced into the mammalian cell by transfection, electroporation or microinjection.
10. The method of embodiment 1, wherein the RNAi molecule is introduced into the mammalian cell by delivering a DNA plasmid or viral vector encoding a short hairpin RNA (shRNA).
11. The method of embodiment 1, wherein apoptosis is induced by exposing the mammalian cells containing the RNAi molecule to ultra violet light.
12. The method of embodiment 1, wherein apoptosis is induced by expression of a pro-apoptotic protein.
13. The method of embodiment 1, further comprising introducing a plasmid DNA or viral expression vector containing a polynucleotide sequence encoding a pro-apoptotic protein into the mammalian cells.
14. The method of embodiment 13, wherein the pro-apoptotic protein is BAX protein.
15. The method of embodiment 1, wherein the RNAi molecule is introduced into the mammalian cell and apoptosis in induced *in vitro.*
16. The method of embodiment 13, wherein the RNAi molecule and the expression vector containing a polynucleotide sequence encoding a pro-apoptotic protein are introduced into the mammalian cell by co-transfection.
17. The method of embodiment 13, wherein the RNAi molecule and the expression vector containing a polynucleotide sequence encoding a pro-apoptotic protein are introduced into an organ or tissue by electroporation, gene-gun, or injection.
18. A method of transfecting a mammalian cell comprising;
   (a) providing a mammalian cell expressing a target gene, wherein the mammalian cell is capable of phagocytosis; and
   (b) exposing the mammalian cell to an apoptotic cell, containing an RNAi molecule capable of downregulating the target gene, under conditions whereby the apoptotic cell is taken up by the mammalian cell.
19. The method of embodiment 18, wherein the RNAi molecule downregulates expression of the target gene in the mammalian cell.
20. The method of embodiment 18, wherein the mammalian cell is an antigen presenting cell.
21. The method of embodiment 18, wherein the mammalian cells are exposed to the apoptotic cells *in vivo* or *in vitro.*
22. A mammalian host cell, comprising
   (a) an RNAi molecule capable of downregulating a target gene; and
   (b) an expression vector capable of expressing a pro-apoptotic protein.
23. The mammalian host cell of embodiment 22, further comprising a specific antigen selected from the group consisting of an autoantigen and a donor antigen.

All references cited in this disclosure are incorporated herein by reference in their entirety.

### REFERENCES

Behlke, M.A. (2006) Progress Towards In Vivo Use of siRNAs. Molecular Therapy 13/4:644-670
Holmgren, L, Szeles, A., Rajnavolgyi, E., Foldman, J., Klein, G., Ernberg, I. and Falk, K.I. (1999) Horizontal Transfer of DNA by the Uptake of Apoptotic Bodies, Blood 93/11:3956-3963.
Li, M., Qian, H., Ichim, T.M., Ge, W-W., Popov, I.A., Rycerz, K., Neu, J., White, D., Zhong, R., and Min, W.-P. (2004) Induction of RNA Interference in Dendritic Cells. Immunologic Research 30/2:215-230.
Zhao H.-F., L'Abbe D., Jolicoeur, N., Wu, M,. Li, Z., Zhenbao, Y., and Shen S-H. (2005) High-Throughput Screening of Effective siRNAs from RNAi Libraries Delivered via Bacterial Invasion, Nature Methods 2/12:967-973.

## Claims

1. A mammalian apoptotic or pre-apoptotic cell for use in treating an autoimmune disorder, wherein the mammalian apoptotic or pre-apoptotic cell comprises a first polynucleotide encoding an autoantigen, a second polynucleotide encoding an RNAi molecule that regulates immunity, and a third polynucleotide encoding a pro-apoptotic protein, wherein one or more expression vectors comprise the first, second, and third polynucleotides.

2. The mammalian cell for use in treating an autoimmune disorder of claim 1, wherein a single expression vector comprises the first, second, and third polynucleotides.

3. The mammalian cell for use in treating an autoimmune disorder of claim 1, wherein separate expression vectors comprise each of the first, second, and third polynucleotides.

4. The mammalian cell for use in treating an autoimmune disorder of any one of the preceding claims, wherein the RNAi molecule comprises a double-stranded RNA (dsRNA), a short interfering RNA (siRNA), or a short hairpin RNA (shRNA).

5. The mammalian cell for use in treating an autoimmune disorder of any one of the preceding claims, wherein the RNAi molecule comprises a short double-stranded RNA molecule (dsRNA) of 19-27 base pairs in length, or a siRNA comprising a short double-stranded RNA molecule (dsRNA) of 19-23 base pairs in length, each strand having a single-stranded overhang of two bases on one end.

6. The mammalian cell for use in treating an autoimmune disorder of any one of the preceding claims, wherein the expressed pro-apoptotic protein is a BAX protein.

7. The mammalian cell for use in treating an autoimmune disorder of claim 6, wherein the BAX protein is encoded by a polynucleotide sequence of SEQ ID NO:3.

8. The mammalian cell for use in treating an autoimmune disorder of any one of the preceding claims, wherein the RNAi molecule that regulates immunity targets CD40.

9. The mammalian cell for use in treating an autoimmune disorder of any one of the preceding claims, wherein the expressed autoantigen is recognized by the immune system of a subject suffering from the autoimmune disorder.

10. The mammalian cell for use in treating an autoimmune disorder of any one of the preceding claims, wherein the one or more expression vector is introduced into the mammalian cell by transfection, viral delivery, electroporation, or microinjection.

11. The mammalian cell for use in treating an autoimmune disorder of any one of the preceding claims, wherein the mammalian apoptotic or pre-apoptotic cell is an apoptotic cell.

12. A composition for use in the treatment of an autoimmune disorder comprising the mammalian cell of claim 1.
